# EUROPEAN PATENT SPECIFICATION

(11) **EP 2 540 315 B1**
(45) Date of publication and mention of the grant of the patent: **30.09.2015**
(21) Application number: 11747344.7
(22) Date of filing: 22.02.2011
(51) Int. Cl.: A61K 45/06, A61K 36/37, A61K 36/60, A61P 1/16, A61P 43/00, A61K 36/27

(54) **USE OF SALACIA EXTRACT FOR CONTROLLING PRODUCTION OF PRIMARY BILE ACID AND SECONDARY BILE ACID FOR THE PROPHYLAXIS OF COLORECTAL CANCER**
VERWENDUNG VON SALACIA EXTRAKT ZUR STEUERUNG DER PRODUKTION PRIMÄRER GALLENSÄURE UND SEKUNDÄRER GALLENSÄURE ZUR VORBEUGUNG VON DARMKREBS
UTILISATION D'EXTRAIT DE SALACIA POUR MAÎTRISE DE LA PRODUCTION D'ACIDE BILIAIRE PRIMAIRE ET D'ACIDE BILIAIRE SECONDAIRE POUR LA PRÉVENTION DU CANCER COLORECTAL

(30) Priority: 25.02.2010 JP 2010040501
(43) Date of publication of application: 02.01.2013
(73) Proprietor: FUJIFILM Corporation, Tokyo 106-8620 (JP)
(72) Inventor: ODA, Yuriko, Ashigarakami-gun Kanagawa 258-8577 (JP); UEDA, Fumitaka, Ashigarakami-gun Kanagawa 258-8577 (JP)
(74) Representative: Morpeth, Fraser Forrest
(86) International application number: PCT/JP2011/053869
(87) International publication number: WO 2011/105383

(56) References cited:
- EP-A1- 2 422 813
- WO-A2-2009/031690
- JP-A- 11 335 285
- JP-A- 2001 253 829
- JP-A- 2006 056 839
- JP-A- 2009 137 899
- US-A1- 2008 220 098
- NAKAMURA YUMIKO ET AL: "Fecal steroid excretion is increased in rats by oral administration of gymnemic acids contained in Gymnema sylvestre leaves", JOURNAL OF NUTRITION, vol. 129, no. 6, June 1999 (1999-06), pages 1214-1222, XP002699933, ISSN: 0022-3166
- BARTRAM H-P ET AL: "EFFECT OF STARCH MALABSORPTION ON FECAL BILE ACIDS AND NEUTRAL STEROLS IN HUMANS POSSIBLE IMPLICATIONS FOR COLONIC CARCINOGENESIS", CANCER RESEARCH, vol. 51, no. 16, 1991, pages 4238-4242, XP002699934, ISSN: 0008-5472
- BENNO, Y. ET AL.: 'Effects of Two alpha-Glucosidase Inhibitors, Acarbose, and BAY-m-1099' INTESTINAL MICROFLORA, CECAL PROPERTIES, BODY- WEIGHT GAINS, SERUM CHOLESTEROL AND SERUM LIPIDS OF RATS, BIFIDOBACTERIA AND MICROFLORA vol. 7, no. 2, 1988, pages 75 - 85
- SHIGEMATSU, N. ET AL.: 'Effect of Administration with the Extract of Gymnema sylvestre R. Br Leaves on Lipid Metabolism in Rats' BIOLOGICAL & PHARMACEUTICAL BULLETIN vol. 24, no. 6, June 2001, pages 713 - 717
- FUSAE TAKAMINE ET AL.: 'Molecular biology of bile acid conversion by celozoic bacteria. Bile acid 7.ALPHA. - dehydration oxidation reaction' JOURNAL OF INTESTINAL MICROBIOLOGY vol. 11, no. 2, 1998, pages 97 - 104
- LI YUHAO ET AL: "Salacia root, a unique Ayurvedic medicine, meets multiple targets in diabetes and obesity", LIFE SCIENCES, vol. 82, no. 21-22, May 2008 (2008-05), pages 1045-1049, ISSN: 0024-3205

## Description

### Technical Field

The present invention relates to a primary bile acid and secondary bile acid generation regulator.

### Background Art

Bile, which plays an important role in digestion and absorption of fat, contains a large amount of bile acids. These bile acids are generated by biosynthesis in the liver using cholesterol as a raw material and secreted from the liver into the biliary tract system as primary bile acids. The primary bile acids predominantly include cholic acid and chenodeoxycholic acid, conjugate with glycine or taurine to form conjugated bile acids, and are secreted from the biliary tract into the intestine. The secreted bile acid, by acting as a surfactant, is involved in digestion and absorption of dietary lipids. The majority of the bile acids in the intestine are reabsorbed from the terminal ileum and then collected in the liver via a portal vein to be reused. Lack of the bile acids results in decreased efficiency of the digestion and absorption of lipids, maldigestion, and insufficient nutrient sources. It is known that when bile acid generation decreases, cholesterol consumption also decreases, which causes an increase in blood cholesterol levels.

Primary bile acids secreted as bile are converted to secondary bile acids by some intestinal bacteria. Secondary bile acids include components such as deoxycholic acid, lithocholic acid, ursodeoxycholic acid and the like, which are not included in primary bile acids, and are suspected to contribute to the onset of colorectal cancer.

Because of this, in order to lower the amount of secondary bile acids in the intestine, development of components having a mechanism of suppressing generation of secondary bile acids and of components having a mechanism of adsorbing secondary bile acids is ongoing.

Because the generation of bile acids is associated with consumption of cholesterol, techniques for reducing blood cholesterol by increasing the amount of bile acids generated have been developed. For instance, Japanese Patent Application Laid-Open (JP-A) No. 2004-051615 discloses a bile acid adsorbent having chitosan-orotic acid salt as an active component. With regard to this bile acid adsorbent, it described that chitosan-orotic acid salt adsorbs and removes the bile acids in the intestinal tract thereby suppressing enterohepatic circulation of bile acids and resulting in promotion of the catabolic reaction from cholesterol to bile acids.

JP-A No. 2001-302529 discloses a bile acid generation promoter containing a tea extract. With regard to this bile acid generation promoter, it describes that a catechin tablet increases a serum bile acid concentration.

Meanwhile, with regard to suppression of the generation of secondary bile acids, techniques focusing attention on intestinal bacteria are known. For instance, Japanese Patent Publication No. 3572103 discloses a secondary bile acid lowering agent having a prescribed galacto oligosaccharide as an active component and describes that this galacto oligosaccharide suppresses the generation of secondary bile acids in the human intestine. JP-A No. 2006-314219 discloses a method for adsorbing bile acids, the method taking advantage of a finding that a certain intestinal bacterial strain has a property of taking up bile acids into the bacterial body.

JP 2009 137899 discloses the use of a pulverized extract of Salacia for reducing toxic intestinal bacteria. WO 2009/031690 discloses the use of a Salacia extract in combination with other plant extracts containing quercetin and resveratol for decreasing body fat and serum cholesterol. It is known from JP 2001 253829, JP 2006 056839 and Bartram H-P et al. Cancer Research, Vol 51, 1991, 4238-4242 that the generation of primary bile acid and suppression of the generation of secondary bile acid can help prevent colorectal cancer. Other relevant art is Benno, Y. et.al., Bifidobacteria and Microflora, vol.7, 1988, pages 75-85, Shigematsu, N. et.al., Biological and Pharmaceutical Bulletin, vol. 24, 2001, pages 713-717, JP 11 335285, Fusae Takamine et.al., Journal of Intestinal Microbiology, vol. 11, 1998, pages 97-104, Nakamura Yukimo et.al., Journal of Nutrition, vol. 129, 1999, pages 1214-1222 and EP 2422813.

### SUMMARY OF INVENTION

### Technical Problem

However, as for generation of bile acids biosynthesized in the living body, that is, primary bile acids, there are no known bile acid generation promoters which are directly involved in the biosynthetic pathway in the living body and enhance the biosynthesis of bile acids. In JP-A No. 2004-051615, the bile acids themselves are adsorbed with the chitosan-orotic acid salt and thus the amount of bile acids available in the living body does not actually increase.

In JP-A No. 2001-302529, a serum cholesterol amount and a serum bile acid amount are measured. However, the measurement does not precisely reflect the state of completely closed enterohepatic circulation and thus the substantive amount of primary bile acids generated actually does not necessarily increase. In addition, because serum bile acids, by their very nature, are elevated in acute hepatitis, cholestasis, or after meals, the amount of bile acids generated per se is not clear.

Furthermore, even though the generation of primary bile acids is closely associated with that of secondary bile acids, all of the techniques described in the documents above only consider either primary bile acids or secondary bile acids. Thus far, a technique for regulating directly the amount of both of these acids is not known.

Accordingly, an object of the present invention is to provide a *Salacia* genus plant extract for use in a prophylactic treatment of an onset of colorectal cancer.

### Solution to Problem

The present invention includes the following modes.
[1] A *Salacia* genus plant extract for use in a prophylactic treatment of an onset of colorectal cancer.
[2] The *Salacia* genus plant extract according to [1], wherein a sucrase 50% inhibition (IC₅₀) of the *Salacia* genus plant extract is from 0.0001 µg/ml to 800 µg/ml.
[3] The *Salacia* genus plant extract according to [1] or [2], wherein the *Salacia* genus plant extract is derived from at least one plant selected from the group consisting of *Salacia reticulata* (*S reticulata*), *Salacia oblonga* (*S.oblonga*), *Salacia prinoides* (*S*. *prinoides*) and *Salacia chinensis* (*S.chinensis*).
[4] The *Salacia* genus plant extract according to [3], wherein the *Salacia* genus plant extract is an extract powder obtained by extracting from the at least one selected from the group consisting of roots and trunks of at least one plant selected from the group consisting of *Salacia reticulata* (*S. reticulata*), *Salacia oblonga* (*S. oblonga*), *Salacia prinoides* (*S*. *prinoides*) and *Salacia chinensis* (*S*. *chinensis*).

### BEST MODE FOR CARRYING OUT THE INVENTION

*A Salacia* genus plant extract for the use of the present invention is a primary bile acid and secondary bile acid generation regulator including an α-glucosidase activity inhibitor component (hereinafter may be referred to simply as "bile acid generation regulator").

Because the bile acid generation regulator for the use of the present invention includes the α-glucosidase activity inhibitor component, it suppresses, while promoting the biosynthesis of bile acids in the living body to enhance the generation of primary bile acids, the generation of secondary bile acids from primary bile acids and reduces the amount of secondary bile acids in spite of the increase in the amount of primary bile acid generated. As a result, the amount of both of primary bile acids and secondary bile acids generated in the living body can be regulated to an appropriate amount for the living body.

In the present invention, to "regulate to an appropriate amount for a living body" means, when the amount after administration of the present agent is compared with the amount before the administration, to increase a ratio of the amount of primary bile acids to the amount of secondary bile acids by having the increased amount of primary bile acid generated in concurrence with the decreased amount of secondary bile acids generated.

As used herein, the term "process" means not only an individual process but also a process in which an expected effect in the process is attained even when the process cannot be clearly distinguished from one or more other processes.

As used herein, a numerical range expressed by using "from A to B" indicates a range including A and B as the minimum value and the maximum value respectively.

When the amount of a component is referred in the present invention, in cases where there are two or more substances corresponding to one component of the composition, unless specified that the amount is an amount of a single substance, the amount of the component means the total amount of the two or more substances.

The present invention is described below.

An α-glucosidase activity inhibitor component in the present invention can be any component as long as it inhibits α-glucosidase present in small intestinal epithelia. Examples thereof include acarbose, voglibose, miglitol, salacinol, kotalanol, deoxynojirimycin, and acteoside. One kind of these may be used singly or two or more kind thereof may be used in combination.

In the case of the extract of *Salacia* genus plant(s), a hot water extract or alcohol extract may be used. One kind of these may be used singly, or two or more kinds thereof may be used in combination of two or more.

*A Salacia* genus plant is a plant of Celastraceae family which grows wild mainly in Sri Lanka, India, and Southeastern Asian area. More specifically, one or more plants selected from *Salacia reticulata* (*S. reticulata*), *Salacia oblonga* (*S. oblonga*), *Salacia prinoides* (*S. prinoides*), *Salacia chinensis* (*S. chinensis*) are used. One obtained by grinding such plant(s), and an extract powder obtained by extracting from edible parts such as roots, trunks, leaves, flowers, fruits, and the like are used. One or more parts may be mixed to be used. More preferably, the extract powder obtained by extracting from the root and/or trunk is used.

The extract powder of *Salacia* genus plant(s) is a product obtained by subjecting the edible part(s) described above to solvent extraction and drying the resulting product. The extraction solvent may be selected from water; alcohol such as methanol or ethanol, or a mixed solvent of water and alcohol(s) or ketone(s) such as acetone. Preferably, water, alcohol, or hydrous alcohol is used. More preferably, hot water or ethanol or hydrous ethanol is used as the extraction solvent. The alcohol concentration of the above-mentioned hydrous alcohol may be, for example, from 30 to 90%, preferably from 40 to 70%. A method of drying includes spray drying, freeze drying and the like, but is not limited thereto.

It is preferred that an α-glucosidase inhibition component in the present invention have a sucrase 50% inhibitory concentration (IC₅₀ value) of from 0.0001 µg/ml to 800 µg/ml. The sucrase 50% inhibitory concentration is more preferably from 0.001 µg/ml to 600 µg/ml, still more preferably from 0.001 µg/ml to 450 µg/ml. The sucrase 50% inhibitory concentration (IC₅₀ value) can be measured by the method described in paragraphs [0009] to [0012] in JP-A No. 2009-249315. For instance, the IC₅₀ value of a component derived from as *Salacia* genus plant(s) is not more than 450 µg/ml.

A bile acid generation regulator may be in the form of any of liquid, solid, powder, and gel. It may be also in the form of solution, tablet, hard capsule, soft capsule, granule or the like.

The content of the α-glucosidase activity inhibitor component in the bile acid generation regulator varies depending on a dosage form or administration mode of the bile acid generation regulator. It can be any amount as long as it is an amount effective for activities of regulating bile acid generation. For instance, when the bile acid generation regulator is in the form of solution, the content of the α-glucosidase activity inhibitor component can be from 90 mass% to 1 mass% , preferably from 73 mass% to 1 mass%, of the total mass of the bile acid generation regulator; when the bile acid generation regulator is in the form of solid, the content of the α-glucosidase activity inhibitor component can be from 90 mass% to 0.5 mass%, preferably from 80 mass% to 1 mass%; of the total mass of the bile acid generation regulator; but the content of the α-glucosidase activity inhibitor component is not particularly restricted. The Dose varies depending on the dosage form and the like. In general, the dose can be from 0.5 mg to 1500 mg once a day, preferably from 2 mg to 800 mg once a day, as the α-glucosidase activity inhibitor component, but is not particularly restricted.

The bile acid generation regulator may contain a pharmaceutically acceptable carrier or any other well known additional component according to its dosage form or administration mode. In cases where it is in the form of solution, examples of a preferred carrier used include an aqueous medium such as water or the like. Examples of a preferred additional component which can be used to form into a solid include a diluent such as crystalline cellulose or magnesium stearate and a bulking agent such as corn starch or alginic acid. Examples of a coating agent which can be used for of tablets, capsules, and granules include shellac, sugar, film coating base material, YEAST WRAP.

In the present invention, in order to improve time-dependent discoloration of *Salacia* genus plant extract powder, it is preferred that calcium carbonate or silicon dioxide is included in an amount of not less than 1% of the mass of the tablet or hard capsule. Furthermore, a low moisture absorption raw material or moisture absorbent utilizable as a food product or food product additive can be used. Preferably, as the low moisture absorption raw materials, cellulose, crystalline cellulose, powdered cellulose, microcrystalline cellulose, lactose, oligosaccharide, sugar alcohol, trehalose, magnesium stearate, calcium stearate or the like is used. As the moisture absorbent, silicates, magnesium carbonate, ferrocyanide, polysaccharide or the like may be used. More preferably, as the low moisture absorption raw material, crystalline cellulose, microcrystalline cellulose or lactose is used. Examples of a compound necessary for forming into a powder, solid formulation or liquid formulation includes erythritol, maltitol, hydroxypropylcellulose, kaolin, talc and the like.

As an administration mode of the bile acid generation regulator, oral administration is preferred. Yet, parenteral administration, for example rectal administration or sublingual administration may be employed.

### EXAMPLES

By way of examples, the present invention is described in detail below. However, the present invention is by no means limited thereto. Unless otherwise noted, "part" is based on mass.

### Example 1

The bile acid biosynthesis promotion by an α-glucosidase activity inhibitor component was checked on the basis of expression (level) of Cyp7a1 in the liver, as described below. Cyp7a1 is an enzyme catalyzing a specific step in the principal bile acid synthesis pathway, the step which is the first stage and rate-limiting step, and is thus known as one of the most important enzymes in the production of bile acids.

Eight-week-old male SD rats were randomly divided into groups of 7 rats. The rats were fed with a solid feed CRF-1 (manufactured by Oriental Yeast Co., Ltd.), which was sterilized by radiation, under usual conditions, and also given using a gastric tube a solid feed in to which the active components described in Table 1 was mixed such that 20 mg/kg/day for each was attained, for 30 days.

As an active component of the sample described in Table 1, the following was used. These components were individually dissolved in water for injection and the resulting solution was administrated as an aqueous solution having a concentration of 10 mg/ml.

Sample B has a *Salacia* genus plant extract as the active component. As the *Salacia* genus plant extract, a *Salacia* extract powder prepared by grinding the root and trunk parts of *Salacia reticulata* (*S. reticulata*) and *Salacia oblonga* (*S. oblonga*) and subjecting the resultant to a process of hot water extraction at 98°C followed by spray drying the resulting liquid, was used.

With respect to the sample, the sucrase IC₅₀. was examined. It was confirmed that it had a sucrase IC₅₀ of not more than 450 µg/ml.

In addition, as other active components which were confirmed not to exhibits activities of inhibiting the a-glucosidase activity, glucose, isomalto oligosaccharide, galacto oligosaccharide, mannan oligosaccharide, indigestible dextrin, and lard were used.

For isomalto oligosaccharide, "PANORUP" from Hayashibara Shoji, Inc., was used and, for galacto oligosaccharide, "Cup Oligo" from Nissin Sugar Manufacturing Co., Ltd. was used. As the indigestible dextrin, a product manufactured by CycloChem Co., Ltd. was used.

After the 30-day feeding period for each sample, the liver was taken out from each rat in each group and all of the mRNAs were then extracted in accordance with a conventional method. The expression level of Cyp7a1 in the liver was measured by carrying out RT-PCR in accordance with a conventional method using all of the extracted mRNAs of each group, and evaluated as a percentage to the expression level in Sample A. The results are shown in Table 1. *: P<0.05

**[Table 1]**

| Sample | Component contained | Expression level of Cyp7a1 mRNA [%] |
|---|---|---|
| A | - | 100 ± 7.3 |
| B | *Salacia* genus plant extract | 134.2 ± 9.1 * |
| C | Glucose | 98.0 ± 4.6 |
| D | Isomalto oligosaccharide | 88.9 ± 10.3 |
| E | Galacto oligosaccharide | 95.7 ± 3.5 |
| F | Mannan oligosaccharide | 102.6 ± 5.7 |
| G | Indigestible dextrin | 94.8 ± 6.0 |
| H | Lard | 86.2 ± 13.7 |

| | | |
|---|---|---|
| * P < 0.05 | | |

As shown in Table 1, in the sample B containing a *Salacia* genus plant extract the expression level of Cyp7a1 significantly increased, when compared with the non-administration group. It is therefore found that the *Salacia* genus plant extract promotes bile acid biosynthesis by increasing the expression level of Cyp7a1 in the liver.

### Example 2

Next, using a *Salacia* genus plant extract as an α-glucosidase activity inhibitor component, the amount of primary bile acids and secondary bile acids generated was measured in feces in the following manner.

Eight-week-old male SD rats were divided into three groups of 10 rats. The rats in these three groups were fed with a solid feed into which the *Salacia* genus plant extract prepared in Example 1 was mixed such that a dose of 0 mg/day/kg, 20 mg/day/kg or 40 mg/day/kg was attained, using a gastric tube for 10 days. A non-administration group was administered with water for injection alone.

After the 30-day administration of each sample, feces were collected for a day and subjected to the measurement. The feces collected were dried using a lyophilizer and their dry weight was measured, and thereafter, the feces were ground with a milling device and extracted with hot ethanol, thereby obtaining a sample for the measurement. The amount of primary bile acids and secondary bile acids was measured using high performance liquid chromatography with a 3α-hydroxysteroid dehydrogenase (3α-HDS) immobilized enzyme column, and fluorescence analysis. The results are shown in Table 2.

**[Table 2]**

| Sample | Doso of *Salacia* genus plant extract (mg/day) | Amount of primary bile acids P (µmol/g) | Amount of secondary bile acid S (µmol/g) | P/S |
|---|---|---|---|---|
| I | 0 | 0.30 ± 0.02 | 0.73 ± 0.03 | 0.41 ± 0.04 |
| J | 20 | 0.44 ± 0.03 | 0.29 ± 0.01 | 1.52 ± 0.16* |
| K | 40 | 0.62 ± 0.01 | 0.37 ± 0.02 | 1.69 ± 0.08* |

| | | | | |
|---|---|---|---|---|
| *P < 0.05 **P < 0.01 | | | | |

As shown in Table 2, in Sample J and Sample K containing a *Salacia* genus plant extract, when compared with a non-administrated group (Sample I), the amount of primary bile acids in the feces increased and concurrently the amount of secondary bile acids in the feces markedly decreased. It is found that a ratio of increase in the primary bile acids generated was larger than that in the secondary bile acids and, thus, the amount of the primary bile acids generated markedly increased. And, at 20 mg/day, the amount of the secondary bile acids was about half or less when compared with that in the non-administration group. Even when the amount of *Salacia* genus plant extract was doubled, the amount of the secondary bile acids decreased markedly when compared with that in non-administration group. With regard to the amount of feces of the rat used in the experiment, there were no significant differences among the groups.

Hence, according to the present invention, the biosynthesis of primary bile acids is directly promoted, and concurrently the generation of the secondary bile acids in feces can be suppressed. As a result, the amount of the primary bile acids and secondary bile acids in the body can be regulated to an appropriate amount for the living body.

## Claims

1. A *Salacia* genus plant extract for use in a prophylactic treatment of an onset of colorectal cancer.

2. The *Salacia* genus plant extract according to claim 1, wherein a sucrase 50% inhibition (IC₅₀) of the *Salacia* genus plant extract is from 0.0001 µg/ml to 800 µg/ml.

3. The *Salacia* genus plant extract according to claim 1, wherein the *Salacia* genus plant extract is derived from at least one plant selected from the group consisting of *Salacia reticulata* (*S reticulata*), *Salacia oblonga* (*S.oblonga*), *Salacia prinoides* (*S. prinoides*) and *Salacia chinensis* (*S.chinensis*).

4. The *Salacia* genus plant extract according to claim 3, wherein the *Salacia* genus plant extract is an extract powder obtained by extracting from the at least one selected from the group consisting of roots and trunks of at least one plant selected from the group consisting of *Salacia reticulata* (*S. reticulata*), *Salacia oblonga* (*S. oblonga*), *Salacia prinoides* (*S*. *prinoides*) and *Salacia chinensis* (*S*. *chinensis*).

## Patentansprüche

1. Extrakt einer Pflanze der Gattung *Salacia* zur Verwendung in einer prophylaktischen Behandlung eines Ausbruchs von Kolorektalkrebs.

2. Extrakt einer Pflanze der Gattung *Salacia* nach Anspruch 1, wobei eine 50%ige (IC₅₀) Succrasehemmung des Extrakts einer Pflanze der Gattung *Salacia* 0,0001 µg/ml bis 800 µg/ml beträgt.

3. Extrakt einer Pflanze der Gattung *Salacia* nach Anspruch 1, wobei der Extrakt einer Pflanze der Gattung *Salacia* von mindestens einer Pflanze, ausgewählt aus der Gruppe bestehend aus *Salacia reticulata* (*S reticulata*), *Salacia oblonga* (*S*. *oblonga*), *Salacia prinoides* (*S*. *prinoides*) und *Salacia chinensis* (*S*. *chinensis*), stammt.

4. Extrakt einer Pflanze der Gattung *Salacia* nach Anspruch 3, wobei der Extrakt einer Pflanze der Gattung *Salacia* ein Extraktpulver ist, das dadurch erhalten wird, dass man aus dem mindestens einen Mitglied der Gruppe bestehend aus Wurzeln und Stämmen von mindestens einer Pflanze, ausgewählt aus der Gruppe bestehend aus *Salacia reticulata* (*S. reticulata*), *Salacia oblonga* (*S. oblonga*), *Salacia prinoides* (*S*. *prinoides*) und *Salacia chinensis* (*S*. *chinensis*), extrahiert.

## Revendications

1. Extrait de plante du genre *Salacia* pour utilisation dans un traitement prophylactique de l'apparition d'un cancer colorectal.

2. Extrait de plante du genre *Salacia* selon la revendication 1, **caractérisé en ce qu'**une concentration inhibitrice à 50 % (CI₅₀) de sucrase de l'extrait de plante du genre *Salacia* est de 0,0001 µg/ml to 800 µg/ml.

3. Extrait de plante du genre *Salacia* selon la revendication 1, **caractérisé en ce que** l'extrait de plante du genre *Salacia* est dérivé d'au moins une plante choisie dans le groupe constitué de *Salacia reticulata* (*S reticulata*), *Salacia oblonga* (*S*.*oblonga*), *Salacia prinoides* (*S*. *prinoides*) et *Salacia chinensis* (*S*. *chinensis*).

4. Extrait de plante du genre *Salacia* selon la revendication 3, **caractérisé en ce que** l'extrait de plante du genre *Salacia* est une poudre d'extrait obtenue par extraction à partir de l'au moins un choisi dans le groupe constitué de racines et de troncs d'au moins une plante choisie dans le groupe constitué de *Salacia reticulata (S. reticulata), Salacia oblonga (S. oblonga), Salacia prinoides (S. prinoides)* et *Salacia chinensis (S. chinensis).*
